# EUROPÄISCHE PATENTSCHRIFT

(11) **EP 2 314 802 B1**
(45) Veröffentlichungstag und Bekanntmachung des Hinweises auf die Patenterteilung: **07.05.2014**
(21) Anmeldenummer: 10008372.4
(22) Anmeldetag: 11.08.2010
(51) Int. Cl.: E05B 1/00, A61L 2/10

(54) **Griff für Flügelelement**
Handle for leaf element
Poignée pour élément de battant

(30) Priorität: 20.10.2009 DE 102009050081
(43) Veröffentlichungstag der Anmeldung: 27.04.2011
(73) Patentinhaber: Dorma GmbH + Co. KG, 58256 Ennepetal (DE)
(72) Erfinder: Brieseck, Bernd, 58840 Plettenberg (DE)

(56) Entgegenhaltungen:
- FR-A1- 2 922 932
- JP-A- 2003 307 049
- JP-U- S5 589 754
- JP-U- S61 177 645

## Beschreibung

Die vorliegende Erfindung betrifft einen Griff für ein Flügelelement mit einer UV-Strahlungsquelle zum Desinfizieren des Griffs, sowie ein Flügelelement mit dem Griff.

Die Flügelelemente von Türen oder Fenstern besitzen Beschläge, die zur Bedienung der Flügelelemente manuell bedienbare Elemente aufweisen. Die manuell bedienbaren Elemente können beispielsweise Türdrücker, Türknäufe oder Griffelemente, wie Fenstergriffe, sein. Im Folgenden wird ein manuell bedienbares Element nur kurz "Griff" genannt, soll aber nicht auf die Bedeutung eines Griffs im engeren Sinn beschränkt sein, sondern umfasst alle denkbaren Elemente, an die manuell zum Öffnen angegriffen werden kann. Derartige Griffe werden in Abhängigkeit des Einsatzorts und -zwecks von einer Vielzahl von Personen bedient.

In den letzten Jahren haben spezifische Probleme bezogen auf Infektionen zugenommen, die durch Kontakt mit manuell bedienbaren Elementen auftreten können. Ist das Flügelelement beispielsweise ein Türblatt einer Tür in einem öffentlichen Gebäude, einer öffentlichen Toilette, einem Krankenhaus, einem Hotel, einem Altenheim oder einer Arztpraxis, können Infektionsprobleme verstärkt auftreten. Die Gefahr einer Infektion kann verringert werden, wenn die Gefahr einer Infektion durch Berührung manuell bedienbarer Beschläge mit bakteriziden Mitteln verringert wird.

Es wurden verschiedene Methoden und Einrichtungen vorgeschlagen, um Beschläge an Flügelelementen zu desinfizieren.

Aus der DE 103 05 142 A1 ist ein Türgriff mit einer Oberfläche bekannt, die eine keimtötende Wirkung besitzt. Die Oberfläche ist mit Substanzen behandelt, die eine bakterizide Wirkung besitzen. Diese Substanzen haben jedoch den Nachteil, dass sie entweder relativ toxisch sind oder ihre keimtötende Wirkung zu gering ist. Bei einem regelmäßigen Gebrauch des manuell bedienbaren Elements ist ein Abrieb der Substanzen festzustellen, wodurch die keimtötende Wirkung nachlässt. Ferner wird vorgeschlagen, das manuell bedienbare Element mit einem Metallkörper zu umhüllen, der nach Abrieb der keimtötenden Oberfläche ausgetauscht werden kann. Dies hat jedoch einen erheblichen Wartungsaufwand zur Folge.

Alternativ wurde vorgeschlagen, ein Flügelelement mit einer UV-Strahlungsquelle zu versehen, die zur bakteriziden UV-Bestrahlung des bedienbaren Elements ausgebildet ist. UV-Strahlen sind sehr gut geeignet, Bakterien, Viren, Pilze etc. sehr schnell abzutöten, ohne dass der Einsatz von Chemikalien nötig wäre.

Allerdings bestrahlt eine UV-Strahlungsquelle immer nur einen Teilbereich eines Griffs. Um einen größeren Bereich zu erreichen, muss eine Mehrzahl von UV-Lampen vorgesehen sein, was den Montageaufwand und die Anfälligkeit erhöht, sowie zu komplexen Anordnungen führt.

Ferner offenbaren die Schriften JP 2003 307049, JP S55 89754 und JP S61 177645 ebenfalls derartige Türgriffe mit einer UV-Strahlungsquelle zum Desinfizieren des Griffs, die in einem Gehäuse angeordnet ist, das einen Mantel aufweist. Dabei ist der Griff zumindest teilweise in einem Innenraum des Gehäuses angeordnet, der durch den Mantel begrenzt ist.

Aufgabe der Erfindung ist, die Probleme des genannten Stands der Technik zu überwinden. Insbesondere soll ein Flügelelement mit Griff und mit integrierter UV-Strahlungsquelle zum Desinfizieren des Griffs vorgeschlagen werden, wobei zumindest nahezu die vollständige Oberfläche des Griffs, die bei seiner Bedienung berührt wird, durch die Desinfektionsvorrichtung desinfizierbar ist. Dabei soll gleichzeitig eine geringe Anfälligkeit der UV-Strahlungsquelle gegeben sein.

Diese Aufgabe wird mit sämtlichen Merkmalen des Anspruches 1 gelöst. Vorteilhafte Weiterbildungen der Erfindung sind in den abhängigen Ansprüchen 2 bis 13 angegeben.

Erfindungswesentlich ist, dass die UV-Strahlungsquelle eine Mehrzahl UV-Strahlung erzeugender Leuchtdioden (im Folgenden auch mit "LED" abgekürzt) aufweist. Leuchtdioden besitzen eine deutlich höhere Robustheit als herkömmliche UV-Strahlungsquellen und damit deutlich geringere Ausfallraten. Dadurch verringert sich der Wartungsaufwand erheblich. Dadurch, dass eine Mehrzahl von LEDs vorgesehen ist, wirkt sich außerdem ein Ausfall einer einzelnen LED weitaus geringer aus, als der Ausfall einer herkömmlichen einzelnen UV-Strahlungsquelle, die einen Totalausfall der gesamten UV-Strahlungsquelle bewirkt.

LEDs haben des Weiteren den Vorteil, dass sie nur sehr wenig Wärme erzeugen, da ihre Lichtausbeute deutlich über der herkömmlicher UV-Strahlungsquellen liegen, und dass sie nicht schlagartig ausfallen.

Vorteilhafterweise ist die Mehrzahl UV-Strahlung erzeugender Leuchtdioden den Griff zumindest teilweise umgebend ausgebildet. Dadurch wird das aus dem Stand der Technik bekannte Problem gelöst, dass nur ein Teilbereich des Griffs durch eine in ein Flügelelement integrierte Desinfektionsvorrichtung desinfiziert wird, während ein anderer Teilbereich stets durch eine Abschattung beispielsweise des Griffs selbst außerhalb eines Wirkbereichs einer UV-Strahlungsquelle liegt.

Erfindungswesentlich ist die Mehrzahl an LEDs in einem Gehäuse angeordnet, das einen Mantel aufweist. Dabei kann der Griff zumindest teilweise in einem Innenraum des Gehäuses angeordnet sein, wobei der Innenraum durch den Mantel begrenzt ist.

Das Gehäuse ist geeignet, die LEDs gegenüber äußeren Einwirkungen zu schützen. Des Weiteren wirkt das Gehäuse nach außen gegenüber UV-Strahlen abschirmend.

Vorteilhafterweise ist der Mantel eine gebogene Leiterbahnplatte, auf der die LEDs angeordnet sind, und die so gekrümmt ist, dass sie einen Hohlzylinder ergibt, auf dessen Mantelinnenfläche sich die LEDs befinden. Dadurch wird die Leiterbahnplatte in einem synergetischen Effekt gleichzeitig als schützender Mantel eingesetzt.

Der Griff kann als Drehgriff ausgebildet sein. Unter Drehgriff wird im Rahmen der Erfindung ein Griff verstanden, der nicht durch Herunterdrücken einer Klinke bedienbar ist, sondern durch ein einfaches Drehen. Derartige Drehgriffe sind vor allem für das Schließen von Türblättern geeignet, die lediglich geringen Anforderungen hinsichtlich mechanischer Widerstandskraft, Einbruchsicherheit, Schallschutz und ähnlichem genügen müssen und deswegen in einer leichtgewichtigen Bauweise ausgeführt sind. Dies können beispielsweise Toilettentüren sein. Wegen der genannten geringen Anforderungen können derartige Drehgriffe mit geringen Abmessungen ausgebildet sein, da zum Öffnen einer leichtgewichtigen Tür nur geringe Kräfte angewendet werden müssen.

Der Griff kann zylinderförmig, insbesondere kreiszylinderförmig, ausgebildet sein. Dies hat den Vorteil, dass der Griff mit geringen Abmessungen platzsparend ausgebildet sein kann, wodurch der Mantel, das Gehäuse und die UV-Strahlungsquelle ebenfalls mit geringen Abmessungen ausgebildet werden kann. Unter zylinderförmig ist im Rahmen der Erfindung auch ein zumindest annähernd scheibenförmiger Griff zu verstehen, wie er bei Toilettentüren üblich ist. Der scheibenförmige Griff kann zur besseren Bedienbarkeit ein Greifelement, wie eine wulstartige Erhebung, aufweisen.

Vorteilhafterweise ist um die LEDs jeweils ein Reflektor vorgesehen, der geeignet ist, eine von der LED erzeugte UV-Strahlung zu reflektieren. Die Reflektoren ermöglichen, dass UV-Strahlung, die von einer LED zunächst in einer vom Griff abgewandten Richtung emittiert wird, zum Griff hin umgelenkt werden kann. Somit erhöht sich ein Verhältnis von ausgesandter UV-Strahlungsmenge zu einer UV-Strahlungsmenge, die auf den Griff trifft. Dadurch können beispielsweise LEDs geringerer Leistung eingesetzt werden, als ohne den Einsatz eines Reflektors nötig wäre, um eine selbe Desinfektionsrate zu erzielen.

Des Weiteren kann der Mantel durch den Einsatz des Reflektors vorteilhafterweise aus einem Material gefertigt sein, das keine hohe UV-Beständigkeit aufweist, denn der Mantel wird durch die Reflektoren gegen UV-Strahlen geschützt. Dies ermöglicht den Einsatz eines kostengünstigen Materials für den Mantel, wie eines herkömmlichen, nicht UV-beständigen Kunststoffs.

Vorteilhafterweise kann ein Innenmantel vorgesehen sein, in dem an den Stellen der LEDs Öffnungen vorgesehen sind, durch die die LEDs zum Innenraum hin UV-Strahlen abgeben können. Vorteilhafterweise ragen die LEDs nicht oder nur gering durch die Öffnungen in den Innenraum. Dadurch sind die LEDs mechanisch geschützt, beispielsweise gegenüber Berührung einer den Griff bedienenden Person.

Nach einer vorteilhaften Variante weist eine Mantelaußenfläche des Mantels eine desinfizierende Oberfläche auf. Die Mantelaußenfläche wird nicht von der UV-Strahlung der LEDs getroffen. Daher ist es von Vorteil, wenn die Mantelaußenfläche selbst desinfizierend ist. Da die Mantelaußenfläche bei einer Bedienung des Griffs nicht berührt werden muss, sondern höchstens selten, beispielsweise versehentlich, von einer den Griff bedienenden Person mit einer Hand berührt wird, sind im Einsatz des Griffs nur wenige Keime auf der Mantelaußenfläche zu erwarten. Daher ist eine Oberfläche, die eine nur langsam wirkende Desinfektion bewirkt, ausreichend. Da die Mantelaußenfläche einer nur sehr geringen mechanischen Belastung durch Berührung von Personen ausgesetzt ist, besteht nicht die Gefahr, dass die desinfizierende Oberfläche verschlissen und abgerieben wird und somit die desinfizierende Wirkung verloren geht.

Unter desinfizierend soll im Rahmen der Erfindung jede Wirkung verstanden werden, die eine keimtötende, bakterizide, viruzide, fungizide oder ähnliche Wirkung ist, wobei die genaue Wirkungsweise oder Intensität der Wirkung für die Erfindung unerheblich ist.

Das Gehäuse kann eine ringförmige Seitenwand aufweisen, die den Mantel mit dem Innenmantel verbindet.

Vorteilhafterweise weist die Seitenwand eine desinfizierende Oberfläche auf. Auch die Seitenwand kann eventuell bei einer manuellen Bedienung des Griffs von der bedienenden Person berührt werden. Daher können auch auf der Seitenwand Keime vorliegen. Da die Seitenwand einer nur sehr geringen mechanischen Belastung durch Berührung von Personen ausgesetzt ist, besteht nicht die Gefahr, dass die desinfizierende Oberfläche verschlissen und abgerieben wird und somit die desinfizierende Wirkung verloren geht.

Zumindest eine der desinfizierenden Oberflächen kann Kupfer und/oder eine desinfizierende Nanokompositoberfläche aufweisen. Kupfer oder Nanokompositoberflächen haben sich als besonders wirksam zur Desinfizierung erwiesen. Da die desinfizierenden Oberflächen selten berührt werden, sind dort nur wenige Keime vorhanden, so dass eine relativ langsame desinfizierende Wirkung zur Desinfizierung genügt.

Vorteilhafterweise sind die LEDs zur Erzeugung von UV-Strahlung mit Wellenlängen zwischen 100 und 380 nm, insbesondere zwischen 150 und 280 nm (UV-C-Strahlung), besonders bevorzugt zwischen 250 und 260 nm geeignet. Bei blauer UV-Strahlung zwischen 250 und 260 nm, insbesondere etwa 254 nm sind Abtötungsraten gegenüber Keimen besonders hoch.

Alternativ kann ein Wellenlängenbereich zwischen 315 und 380 nm, der mit UV-A bezeichnet wird, vorteilhaft zur Desinfizierung sein.

Gelöst wird die Aufgabe der Erfindung des Weiteren mit sämtlichen Merkmalen des Patentanspruchs 13. Vorteilhafte Aus- und Weiterbildungen des Flügelelements sind in dem abhängigen Anspruch 14 angegeben.

Nach einer vorteilhaften Ausführungsform ist zumindest eine Steuerung vorgesehen, die geeignet ist, eine Aktivität der LEDs zu steuern.

In Verbindung mit der Steuerung sind vorteilhafterweise Parameter, wie UV-Strahlungsdauer, -intensität und -wellenlänge so einstellbar, dass ein Desinfizieren des Griffs vorbestimmt erfolgen kann.

Nach einem Aspekt der Erfindung sind eine Energieversorgungseinheit, wie ein Stromnetzteil, und elektrische Leitungen zur Stromversorgung der Steuerung, der LEDs und möglicher weiterer Komponenten vorgesehen.

Vorzugsweise ist ein Sensor vorgesehen, der die Anwesenheit einer Person in der Nähe des Flügelelements detektiert. Dies hat den Vorteil, dass die LEDs nicht aktiviert sind und/oder eine Aktivität der LEDs unterbrochen wird, wenn sich eine Person in der Nähe des Griffs befindet. Somit wird eine Verletzungsgefahr durch UV-Strahlung ausgeschlossen.

Es kann ein Mechanismus vorgesehen sein, der unmittelbar nach dem Zufallen der Tür oder währenddessen einen Desinfektionsvorgang in Gang setzt. Dadurch findet unmittelbar nach jeder Bedienung des Flügelelements ein Desinfizieren des Griffs statt. Dies hat den Vorteil, dass der Griff unmittelbar nach jeder Berührung desinfiziert wird und sich Keime daher nicht erst vermehren können. Außerdem entfernt sich im Normalfall die Person, die die Tür bedient hat, nach einem Durchschreiten von der Tür, so dass die Desinfektion durch UV-Bestrahlung erfolgen kann, ohne dass eine Person dadurch geschädigt werden kann, bzw. ohne dass ein Desinfektionsschritt wegen der Nähe der Person unterbleiben muss.

Alternativ ist es auch als vorteilhaft vorstellbar, dass die Steuerung so eingestellt ist, dass ein Desinfizieren automatisch in regelmäßigen zeitlichen Abständen durchgeführt wird.

Des Weiteren ist eine vorteilhafte Variante, dass eine Zähleinrichtung vorgesehen ist, die die Anzahl der Bedienungen des Griffs oder die Anzahl der Bedienungen des Flügelelements bestimmt. Die Steuerung kann nach einer vorbestimmten Anzahl von Bedienungen ein Desinfizieren des Griffs mittels der LEDs aktivieren. Die Anzahl der Bedienungen ist vorteilhafterweise manuell leicht einstellbar und kann vorzugsweise in Abhängigkeit von diversen Parametern, wie der Tageszeit oder einer Infektionsgefahrstufe, programmiert werden.

Weitere, die Erfindung verbessernde Maßnahmen werden nachstehend gemeinsam mit der Beschreibung eines bevorzugten Ausführungsbeispiels der Erfindung anhand der Figuren näher dargestellt.

Dabei zeigen:
- Fig. 1:: eine perspektivische Gesamtansicht eines schematisch dargestellten erfindungsgemäßen Griffs im montierten Zustand,
- Fig. 2:: eine perspektivische Ansicht eines Griffs ohne Innenmantel und
- Fig. 3:: eine perspektivische Teilansicht eines Griffs.

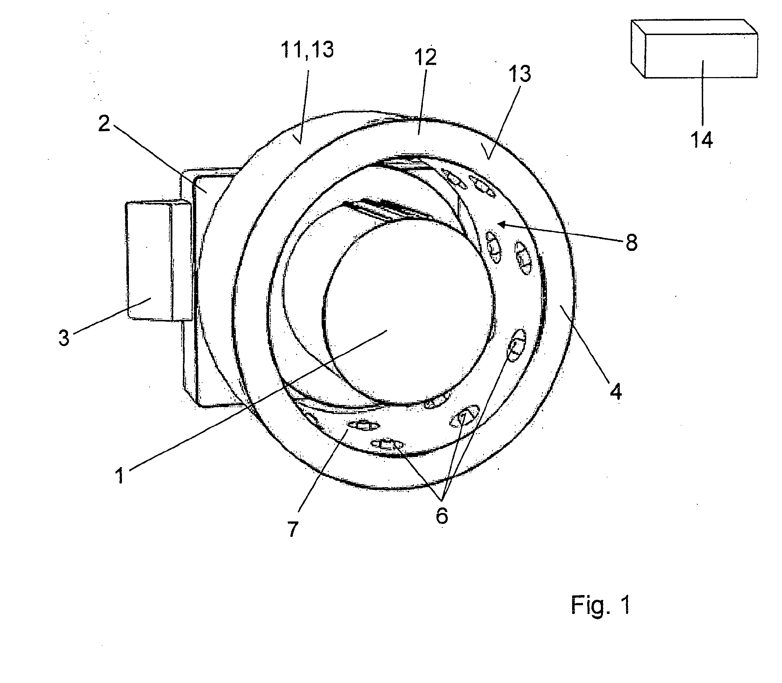

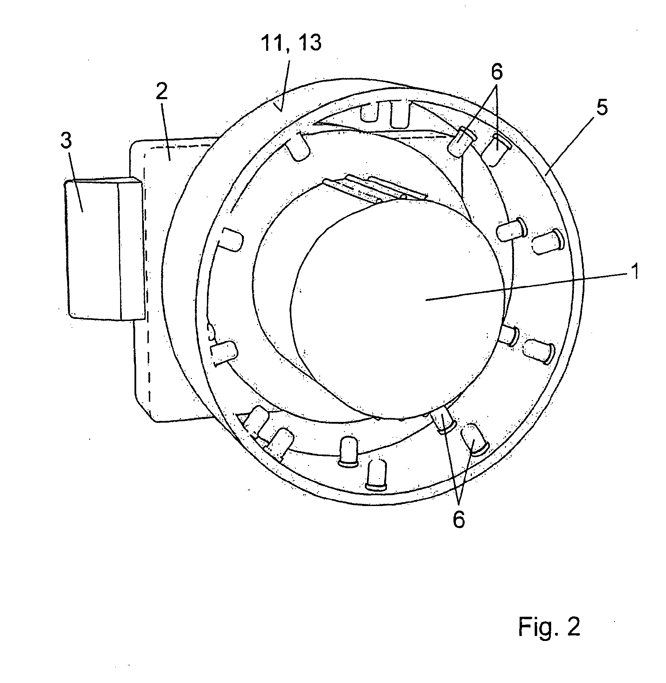

Ein erfindungsgemäßer Griff 1, der gemäß des Ausführungsbeispiels als Toilettentürgriff 1 ausgebildet ist, ist an einem Flügelelement 2, das in diesem Beispiel eine Toilettentür 2 ist, die in Fig. 1 und 2 nur schematisch als Ausschnitt eines Türblatts 2 dargestellt ist, drehbar gelagert. Der Toilettentürgriff 1 ist als kreiszylinderförmiger Drehgriff 1 ausgebildet, d.h. lediglich durch ein Drehen des Griffs 1 kann ein Verschlussriegel 3 bewegt werden und dadurch die Toilettentür 2 verriegelt und entriegelt werden.

Um den Griff 1 ist ein Gehäuse 4 angeordnet, das einen hohlzylinderförmigen Mantel 5 umfasst, auf dem UV-Strahlung emittierende Leuchtdioden 6 angeordnet sind. Der Mantel 5 wird durch eine aufgerollte Leiterbahnenplatte gebildet, auf der die Leuchtdioden 6 über Leiterbahnen (nicht dargestellt) elektrisch verbunden angeordnet sind. Ein Innenmantel 7 ist konzentrisch zu dem Mantel 5 in einem Innenraum 8, der durch den Mantel 5 begrenzt wird, angeordnet. In Öffnungen 9 des Innenmantels 7 ragen die LEDs 6.

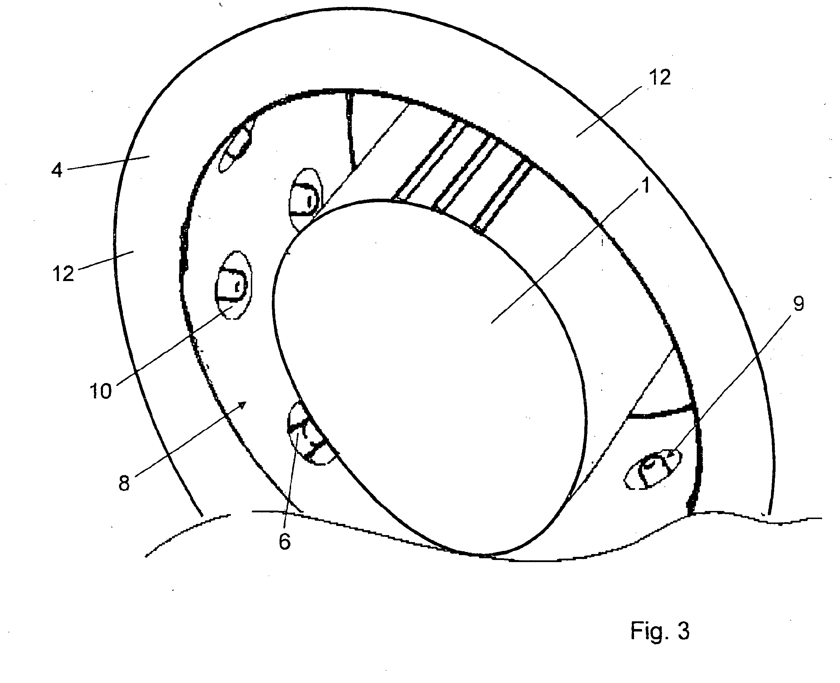

In einer Variante des Ausführungsbeispiels (Fig. 3) ist um jede LED 6 ein Reflektor 10 angeordnet, der von der LED 6 ausgesandtes UV-Licht in Richtung des Griffs 1 verstärkt.

Der Mantel 5 ist in diesem Ausführungsbeispiel höher als der Drehgriff 1 ausgebildet, wodurch der Drehgriff 1 vollständig im Innenraum 8 des Mantels 5 und somit auch innerhalb des Gehäuses 4 angeordnet ist. Alternativ kann der Mantel 5 aber auch niedriger ausgebildet sein, so dass der Mantel 5 nur geringfügig höher als der Griff 1, gleichhoch oder sogar niedriger als der Griff 1 ausgebildet ist. Je nachdem wie von den LEDs 6 ausgestrahlte UV-Strahlen fokussiert oder weit aufgefächert sind, kann in Kombination mit den Reflektoren 10 eine für den jeweiligen Anwendungsfall gewünschte geometrische Ausbildung des Gehäuses 4 und des Griffs 1 vorgesehen sein. Im vorliegenden Beispiel sind zwei Reihen an LEDs 6 vorgesehen. Ein flacherer Griff 1 kann mit einer geringeren Anzahl an LEDs 6 desinfiziert werden, beispielsweise mit nur einer Reihe an LEDs 6.

Eine Mantelaußenfläche 11 des Mantels 5 und eine Oberfläche einer Seitenwand 12, die den Mantel 5 und den Innenmantel 7 miteinander verbinden, sind als Kupferoberfläche 13 ausgebildet. Die Mantelaußenfläche 11 wird von dem der LEDs 6 ausgesandtem UV-Licht nicht erreicht und kann somit auch nicht dadurch desinfiziert werden. Da Kupfer keimtötend wirkt, werden auf der Mantelaußenfläche 11 vorhandene Keime, Bakterien, Pilze und ähnliches zwar langsamer als durch UV-Licht, aber wirkungsvoll abgetötet. Das gleiche gilt für die Oberfläche der Seitenwand 12. Daher ist in diesem Beispiel auch die Oberfläche der Seitenwand 12 als Kupferoberfläche 13 ausgebildet. Alternativ sind andere selbstdesinfizierende Oberflächen 13, wie selbstdesinfizierende Nanokompositoberflächen denkbar.

Die Leuchtdioden 6 des Beispiels senden im Betrieb blaues UV-Licht mit einer Wellenlänge von etwa 254 nm aus, das somit sogenanntes UV-C-Licht darstellt, das bei dieser Wellenlänge besonders hohe Abtötungsraten erzeugt. Außer UV-C-Licht im Bereich von 100 bis 280 nm ist beispielsweise auch UV-A-Licht im Bereich von 315 bis 380 nm zum Desinfizieren geeignet. Des Weiteren ist selbstverständlich auch UV-B-Strahlung zwischen 280 und 315 nm geeignet. In einer Variante umfasst die Mehrzahl von LEDs 6 unterschiedliche Gruppen an LEDs, die UV-Licht in unterschiedlichen Wellenlängen abstrahlen. So lassen sich gezielt unterschiedliche Keime o.ä., die gegenüber speziellem UV-Licht empfindlich sind, abtöten.

Durch regelmäßiges Aktivieren der Mehrzahl von LEDs 6 wird der Griff 1 jedesmal desinfiziert und eine Keimausbreitung und -vermehrung wirksam unterdrückt. In diesem Beispiel wird die Mehrzahl an LEDs 6 in regelmäßigen zeitlichen Abständen, wie beispielsweise 10 min, für einige Sekunden aktiviert. Je nach Einsatzort, Tageszeit und weiteren Faktoren können die LEDs 6 auch mit kürzeren und längeren Zeitabständen oder mit einer kürzeren oder längeren Aktivierungsdauer eingesetzt werden.

Alternativ können die LEDs 6 aufgrund einer durch eine Zähleinrichtung (nicht dargestellt) bestimmte Anzahl von Türbedienungen oder anderweitige Kriterien mittels einer Steuerung (nicht dargestellt) aktiviert und deaktiviert werden.

Befindet sich eine Person in der Nähe des Griffs 1, detektiert dies ein Sensor 14, und die LEDs 6 werden deaktiviert.

LEDs 6, Sensor 14 und Steuerung sind mit einer nicht in den Figuren dargestellten Stromversorgung versehen.

Ein erfindungsgemäßer Griff 1 mit LEDs 6 kann auf einer oder auf beiden Seiten eines Flügelelements 2 vorgesehen sein.

Die Erfindung ist nicht auf Drehgriffe 1 an Toilettentüren 2 beschränkt, sondern kann auf alle Griffe 1 an Flügelelementen 2 übertragen werden.

### Bezugszeichenliste

- 1: Griff, Toilettentürgriff, Drehgriff
- 2: Flügelelement, Türblatt, Toilettentür
- 3: Verschlussriegel
- 4: Gehäuse
- 5: Mantel, Leiterbahnplatte
- 6: UV-Strahlung emittierende Leuchtdiode, LED
- 7: Innenmantel
- 8: Innenraum
- 9: Öffnung
- 10: Reflektor
- 11: Mantelaußenfläche
- 12: Seitenwand
- 13: Desinfizierende Oberfläche, Kupferoberfläche, Nanokomposit- oberfläche
- 14: Sensor

## Patentansprüche

1. Griff (1) für ein Flügelelement (2), insbesondere für ein Türblatt (2), und eine UV-Strahlungsquelle zum Desinfizieren des Griffs (1), wobei die UV-Strahlungsquelle in einem Gehäuse (4) angeordnet ist, das einen Mantel (5) aufweist, wobei der Griff (1) zumindest teilweise in einem Innenraum (8) des Gehäuses (4) angeordnet ist, der durch den Mantel (5) begrenzt ist, **dadurch gekennzeichnet, dass** die UV-Strahlungsquelle eine Mehrzahl von Leuchtdioden (6) enthält, die geeignet sind, UV-Strahlen auszusenden.

2. Griff (1) und UV-Strahlungsquelle nach Anspruch 1, **dadurch gekennzeichnet, dass** die Mehrzahl von Leuchtdioden (6) den Griff (1) zumindest teilweise umgebend angeordnet ist.

3. Griff (1) und UV-Strahlungsquelle nach Anspruch 2, **dadurch gekennzeichnet, dass** der Mantel (5) zylinderförmig ausgebildet ist.

4. Griff (1) und UV-Strahlungsquelle nach Anspruch 1 oder 3, **dadurch gekennzeichnet, dass** der Mantel (5) durch eine Leiterbahnplatte gebildet ist, auf der die Leuchtdioden (6) angeordnet sind.

5. Griff (1) und UV-Strahlungsquelle nach Anspruch 1 oder 2, **dadurch gekennzeichnet, dass** im Innenraum (8) konzentrisch zu dem Mantel (5) ein Innenmantel (7) vorgesehen ist, der Öffnungen (9) aufweist, in die zumindest teilweise die Leuchtdioden (6) ragen.

6. Griff (1) und UV-Strahlungsquelle nach einem oder mehreren der vorhergehenden Ansprüche, **dadurch gekennzeichnet, dass** um Leuchtdioden (6) Reflektoren (10) vorgesehen sind, die geeignet sind, eine von den Leuchtdioden (6) erzeugte UV-Strahlung zu reflektieren.

7. Griff (1) und Strahlungsquelle nach einem oder mehreren der Ansprüche 1 bis 6, **dadurch gekennzeichnet, dass** der Mantel (5) eine Mantelaußenfläche (11) aufweist und die Mantelaußenfläche (11) eine desinfizierende Oberfläche (13) aufweist.

8. Griff (1) und Strahlungsquelle nach einem oder mehreren der Ansprüche 1 bis 7, **dadurch gekennzeichnet, dass** das Gehäuse (4) eine Seitenwand (12) aufweist, die den Mantel (5) und den Innenmantel (7) miteinander verbindet.

9. Griff (1) und Strahlungsquelle nach Anspruch 8, **dadurch gekennzeichnet, dass** die Seitenwand (12) eine desinfizierende Oberfläche (13) besitzt.

10. Griff (1) und Strahlungsquelle nach einem oder mehreren der Ansprüche 7 bis 9, **dadurch gekennzeichnet, dass** zumindest eine desinfizierende Oberfläche (13) Kupfer und/oder eine desinfizierende Nanokompositoberfläche aufweist.

11. Griff (1) und Strahlungsquelle nach einem oder mehreren der vorhergehenden Ansprüche, **dadurch gekennzeichnet, dass** zumindest ein Teil der Leuchtdioden (6) geeignet ist, UV-Strahlung im Bereich von UV-A-Strahlung und/oder UV-C-Strahlung zu erzeugen.

12. Flügelelement (2), insbesondere Türblatt (2) oder Fensterblatt, mit einem Griff (1) und Strahlungsquelle nach einem oder mehreren der Ansprüche 1 bis 11.

13. Flügelelement (2) nach Anspruch 12, **dadurch gekennzeichnet, dass** das Flügelelement (2) einen Sensor (14) aufweist, der geeignet ist, eine Person in einem Wirkbereich der Leuchtdioden (6) zu detektieren.

## Claims

1. A handle (1) for a leaf element (2), in particular for a door leaf (2), and a UV-radiation source for disinfecting the handle (1), wherein the UV-radiation source is disposed in a housing (4) which has a cladding (5), wherein the handle (1) is disposed, at least partially, in an inner space (8) of the housing (4), which space is delimited by the cladding (5), **characterized in that** the UV-radiation source comprises a plurality of light-emitting diodes (6), which are suitable to emit UV-rays.

2. The handle (1) and the radiation source according to claim 1, **characterized in that** the plurality of light emitting diodes (6) are disposed to surround the handle (1) at least partially.

3. The handle (1) and the radiation source according to claim 2, **characterized in that** the cladding (5) is configured to be cylindrical.

4. The handle (1) and the radiation source according to claim 1 or 3, **characterized in that** the cladding (5) is formed by means of a printed circuit board on which the light emitting diodes (6) are disposed.

5. The handle (1) and the radiation source according to claim 1 or 2, **characterized in that** an inner cladding (7), which has openings (9), into which the light emitting diodes (6) project at least partially, is provided in the inner space (8) concentrically with regard to the cladding (5).

6. The handle (1) and the radiation source according to one or more of the preceding claims, **characterized in that** reflectors (10), which are suitable to reflect a UV-radiation generated by the light emitting diodes (6), are provided around the light emitting diodes (6).

7. The handle (1) and the radiation source according to one or more of the claims 1 to 6, **characterized in that** the cladding (5) features an exterior cladding surface (11) and **in that** the exterior cladding surface (11) features a disinfecting surface (13).

8. The handle (1) and the radiation source according to one or more of the claims 1 to 7, **characterized in that** the housing (4) features a lateral wall (12), which interconnects the cladding (5) and the inner cladding (7).

9. The handle (1) and the radiation source according to claim 8, **characterized in that** the lateral wall (12) includes a disinfecting surface (13).

10. The handle (1) and the radiation source according to one or more of the claims 7 to 9, **characterized in that** at least one disinfecting surface (13) features copper and/or a disinfecting composite nano-surface.

11. The handle (1) and the radiation source according to one or more of the preceding claims, **characterized in that** at least one portion of the light emitting diodes (6) is suitable to generate UV-radiation in the range of UV-A-radiation and/or UV-C-radiation.

12. A leaf element (2), in particular a door leaf (2) or a window leaf with a handle (1) and a radiation source according to one or more of the claims 1 to 11.

13. The leaf element (2) according to claim 12, **characterized in that** the leaf element (2) features a sensor (14) which is suitable to detect a person in an effective range of the light emitting diodes (6).

## Revendications

1. Poignée (1) pour un élément à vantail (2) tout particulièrement pour un vantail de porte (2), et une source de rayonnement UV pour désinfecter la poignée (1), la source de rayonnement UV étant agencée dans un boîtier (4), qui présente un gainage (5), la poignée (1), au moins partiellement, étant agencée dans un espace intérieur (8) du boîtier (4), lequel espace est délimité par l'intermédiaire du gainage (5), **caractérisée en ce que** la source de rayonnement UV comporte de nombreuses diodes électroluminescentes (6), qui sont aptes à émettre des rayons UV.

2. Poignée (1) et source de rayonnement selon la revendication 1, **caractérisées en ce que** les nombreuses diodes électroluminescentes (6) sont agencées, au moins partiellement, de façon a entourer la poignée (1).

3. Poignée (1) et source de rayonnement selon la revendication 2, **caractérisées en ce que** le gainage (5) est aménagé de façon cylindrique.

4. Poignée (1) et source de rayonnement selon la revendication 1 ou 3, **caractérisées en ce que** le gainage (5) est constitué par l'intermédiaire d'un circuit conducteur sur lequel les diodes électroluminescentes (6) sont agencées.

5. Poignée (1) et source de rayonnement selon la revendication 1 ou 2, **caractérisées en ce que**, dans l'espace intérieur (8), un gainage intérieur (7) est prévu de façon concentrique par rapport au gainage (5) et présente des ouvertures (9), dans lesquelles les diodes électroluminescentes (6) font saillie, au moins partiellement.

6. Poignée (1) et source de rayonnement selon l'une ou plusieurs des revendications précédentes, **caractérisées en ce que** des réflecteurs (10), qui sont aptes à réfléchir un rayonnement UV émis par les diodes électroluminescentes (6), sont prévus autour des diodes électroluminescentes (6).

7. Poignée (1) et source de rayonnement selon l'une ou plusieurs des revendications 1 à 6, **caractérisées en ce que** le gainage (5) présente une surface de gainage extérieur (11) et **en ce que** la surface de gainage extérieur (11) présente une surface désinfectante (13).

8. Poignée (1) et source de rayonnement selon l'une ou plusieurs des revendications 1 à 7, **caractérisées en ce que** le boîtier (4) présente une paroi latérale (12) qui relie le gainage (5) et le gainage intérieur (7) l'un à l'autre.

9. Poignée (1) et source de rayonnement selon la revendication 8, **caractérisées en ce que** la paroi latérale (12) possède une surface désinfectante (13).

10. Poignée (1) et source de rayonnement selon l'une ou plusieurs des revendications 7 à 9, **caractérisées en ce qu'**au moins une surface désinfectante (13) présente du cuivre et/ou une surface désinfectante en composite nano.

11. Poignée (1) et source de rayonnement selon l'une ou plusieurs des revendications précédentes, **caractérisées en ce qu'**au moins une partie des diodes électroluminescentes (6) est aptes à générer un rayonnement UV dans la plage de rayonnement UV-A et/ou la plage de rayonnement UV-C.

12. Elément à vantail (2) en particulier un vantail de porte (2) ou vantail de fenêtre avec une poignée (1) et source de rayonnement selon l'une ou plusieurs des revendications 1 à 11.

13. Elément à vantail (2) selon la revendication 12, **caractérisé en ce que** l'élément à vantail (2) présente un capteur (14) qui est aménagé pour détecter une personne dans une zone d'actionnement des diodes électroluminescentes (6).
